(19) Europäisches Patentamt
European Patent Office
Office européen des brevets

(11) **EP 2 352 430 B1**

(12) **EUROPEAN PATENT SPECIFICATION**

(45) Date of publication and mention
of the grant of the patent:
**29.05.2013 Bulletin 2013/22**

(51) Int Cl.:
***A61B 5/16*** *(2006.01)*  ***A61B 5/00*** *(2006.01)*

(21) Application number: **09782081.5**

(22) Date of filing: **21.08.2009**

(86) International application number:
**PCT/EP2009/060830**

(87) International publication number:
**WO 2010/020691 (25.02.2010 Gazette 2010/08)**

(54) **MEASURING AND MONITORING MENTAL CAPACITY**

MESSEN UND ÜBERWACHEN VON MENTALER LEISTUNGSFÄHIGKEIT

MESURE ET SURVEILLANCE DE CAPACITÉ MENTALE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **21.08.2008 EP 08162778**
**19.03.2009 EP 09155635**

(43) Date of publication of application:
**10.08.2011 Bulletin 2011/32**

(73) Proprietor: **ULL METER A/S**
**2900 Hellerup (DK)**

(72) Inventor: **BALLEGAARD, Søren**
**DK-2900 Hellerup (DK)**

(74) Representative: **Inspicos A/S**
**Kogle Allé 2**
**P.O. Box 45**
**2970 Hørsholm (DK)**

(56) References cited:
**WO-A-2006/092146  WO-A-2007/000030**
**WO-A-2008/028976  US-A1- 2002 005 784**

**Description**

FIELD OF THE INVENTION

The present invention relates to the field of assessment of mental performance capacity in humans. In particular, the present invention relates to methods which enable an accurate determination of an individual's present capacity to manage complex mental challenges, cognitive as well as emotional. Further, the invention also on a more general scale relates to general performance assessments for groups and individuals that are to perform a task which includes psychological elements but may also include physical requirements.

BACKGROUND OF THE INVENTION

Whereas it is relatively easy to gauge the physical fitness, there is today no effective and simple means of determining the "psychological fitness" of a person - and as a consequence of this, there is not any way of determining the "overall fitness" either, since the psychological aspects are normally a significant part of the complexity of a given task. This is in spite of the fact that many situations in daily professional life require that both the physical and psychological side of a person or group of person are above some (yet undefined) threshold value in order for the person to perform under a given set of circumstances.

However, the psychological side of human performance is still to a very large degree attributed to "chance events" instead of being properly and quantitatively managed as one does with the physical side.

OBJECT OF THE INVENTION

The invention is defined in the independent claims and their corresponding dependent claims.

It is an object of the invention to provide methods that assists individuals in estimating their capacity for conducting work load, including a future brain work load. It is also an objective to provide individuals and groups with the possibility to train/maintain/improve the psychological fitness measure through training and/or treatment.

DISCLOSURE OF THE INVENTION

The present inventor has discovered that it is possible to quantify the working capacity of the brain in very much the same way in which the working capacity of the heart is determined when measuring the physical fitness based on oxygen consumption during various degrees of physical exercise. A further finding is that the working capacity of the brain is reflected in overall performance.

The physical fitness index is determined as the maximum oxygen consumption rate (measured in ml/min) per kg of body weight and this can be done very accurately using equipment which measures the $CO_2$ output from a person under exercise. In practice, the physical fitness index is, however, often determined by measuring the heart rate as surrogate for oxygen consumption measurements: the heart rate is determined under resting conditions, under varying degrees of workload as well as in a period after hard exercise. A fit person will have a low resting heart rate, the ability to withstand increasing loads of exercise without reaching the maximum heart rate at an early stage during the increases in workload, and, importantly, the ability to revert to the resting heart rate within a short while after termination of hard exercise. So, the heart's behaviour in response to physical work is an accurate indicator of the physical capacity of an individual.

Surprisingly, it has been found by the present inventor that the psychological performance capacity in humans behaves in a manner similar to the physical performance capacity. Instead of using the oxygen consumption or a surrogate thereof as measured variable as is done when determining physical fitness, the level of sympathetic tone or a surrogate thereof can be used as the measured variable.

It has during a series of pilot experiments been found by the present inventor that there is a tight correlation between a person's psychological work capacity and a "psychological fitness index", PFI, where persons having a high PFI are characterised by a low resting sympathetic tone, the ability to withstand increasing psychological workload without reaching a maximum sympathetic tone at an early stage during the increase, and, importantly, the ability to revert to the low resting sympathetic tone within a short while after termination of the psychological workload.

Further, as is the case with the physical fitness index, which may be improved by physical training, it has proven possible to increase the psychological working capacity in individuals by using appropriate training. In this context it is noteworthy that one factor which has been found to be decisive for the "psychological" fitness is an individual's level of mental stress - previous work by the present inventor has shown that psychological stress *i.a.* has the consequence that the resting level of sympathetic tone is increased, meaning that the difference between the maximum sympathetic tone and the resting level is decreased - put in simple words, this seems to mirror the fact that the brain's capacity is "occupied" (as evidenced by the high resting level), and the further consequence is that the capacity for increases in psychological workload is decreased to lesser or higher degree in the psychologically stressed individual.

Similarly, the present invention can be used to distinguish between transient stress, which improves overall performance and the persistent stress, which has the opposite effect.

With respect to sports, stress may be productive and improve performance, but can also deteriorate performance. It is essential to distinguish between two forms of stress: Transient and persistent. Transient stress is the physiological state of preparedness, a state which is automatically induced in the body through neural/hormonal signals from the brain when one perceives or anticipates a challenge or a threat. Thus, it serves as a mechanism which increases performance through: sharpened senses; intensified ability to coordinate input, process information and react accordingly; improved coordination between brain and body functions; increased mental, emotional and physical flexibility in response to changes in circumstances or premises; improved durability in response to prolonged stress exposure; increased emotional tolerance towards challenges, and optimal restitution capacity. The level of stress depends on the balance between the individual expectancies of the outcome of the stimulus and the resources available. When the challenge or threat is over, homeostasis is re-established. This dynamic process of adaption is called allostasis, including an initial activation and a subsequent restitution.

In contrast, persistent stress might be regarded as a dysfunction of the neural/hormonal processes of the brain, and it is associated with decreased performance due to deteriorating of the characteristics of transient stress. This loss of the ability of adaptation, i.e. loss of allostasis, is also called the allostatic load.

It is believed that the present invention provides the first effective means to objectively determine the status of an individual's psychological work capacity, to monitor the same and consequently also to improve it by e.g. instigating measures that reduce the individual's level of permanent psychological stress.

The implications are several. First of all it becomes possible to objectively determine whether a person is sufficiently fit for a given task. In military operations, certain professional sports and other situations where the "psychological fitness" of the persons acting is of vital or high importance, it becomes possible to include this as a further, objectively determined, parameter in addition to physical fitness, technical skills and other parameters normally used when deciding on team composition, training regimen, etc.

Hence, in one general aspect, the present invention relates to a method for determining the capacity for psychological workload in an individual, comprising

(a) determining the level of sympathetic tone in said individual under resting conditions ($\alpha$),
(b) determining the level of sympathetic tone ($\beta$) in said individual under a defined psychological workload ($W_p$),
(c) determining the time ($\Delta t$) it takes said individual to revert from the sympathetic tone level $\beta$ under the defined psychological workload $W_p$, to the sympathetic tone level $\alpha$ under resting conditions, and
(d) expressing the capacity for psychological workload as a function of $\beta$, $\alpha$ and $\Delta t$.

In one embodiment, the present invention relates to a method for determining the capacity for psychological workload ($C_{pw}$) in an individual, comprising

(a) determining the level of sympathetic tone in said individual under resting conditions ($\alpha$),
(b) determining the level of sympathetic tone ($\beta$) in said individual under a defined psychological workload ($W_p$),
(c) determining the time ($\Delta t$) it takes said individual to revert from the sympathetic tone level $\beta$ under the defined psychological workload $W_p$, to the sympathetic tone level $\alpha$ under resting conditions, and
(d) calculating the psychological workload capacity according to the formula I:

$$C_{pw} = (\beta - \alpha) / (W_p \times \Delta t) \text{ or any monotone function thereof.}$$

The "capacity for psychological workload" (used interchangeably with the expression "psychological fitness" and "PFI") denotes the ability of a person to manage demanding and complex psychological challenges, that is, a fitness which involves mental, cognitive, emotional, social, nutritional, physical aspects and thus represent an indicator for the overall performance of the individual (e.g. as indicated by improved brain-body coordination, which will be evidenced by an improved physical performance). The psychological fitness may be measured "internally", *i.e.* for one person relative to an arbitrary index value (cf. below) measured initially according to the present invention, or comparatively, where the psychological fitness is determined against at least one known psychological challenge which has a standardized assigned value for $W_p$ (cf. below).

This overall fitness indicator is significantly correlated to stress, and this connection it is important to distinguish between two different aspects of stress: transitional stress and persistent stress, cf. above.

US 2002/0005784 discloses a system and method for predicting human cognitive performance. Convenient methods for determining the level of stress or the sympathetic tone in an individual are disclosed in WO 2005/084529, WO 2006/092146, and WO 2008/028976, as well as in US provisional patent application No: 61/036,385. These patent applications generally deal with methods for determination of the level of psychological stress in humans and animals. The determinations have been obtained by using measurements of the absolute or relative pain threshold in locations in or on the body where the pain perception is dependent on the sympathetic tone. The methods for sympathetic tone determination (and, under some circumstances, the stress level, warning systems sensitivity or other surrogates) which are disclosed in these patent applications are all applicable and preferred for the purposes of the present invention. The values of $\beta$ and $\alpha$ can accordingly be expressed as functions of the force used to exert a stimulus in a sympathetic tone dependent location so as to reach the threshold of pain. Conveniently, $\beta$ and $\alpha$ are mathematically derived from the pressure force necessary to elicit pain in sympathetic tone dependent location on the body. The mathematic derivation may be a logarithmic scale expressing an inverse correlation between the sympathetic tone and the nociception threshold value in such a sympathetic tone dependent location.

In particular, $\beta$ and $\alpha$ can be measured with an algometer (Nussbaum, E. L., Downes, L. "Reliability of clinical pressure-pain algometric measurements obtained on consecutive days". Phys Therapy 78, 160-169 (1998)). According to the present invention an algometer was used as follows:

In the example, algometer readings were hidden until the measurement was completed in order to blind the subject and researcher. For analysis, the mean of two consecutive measurements was used.

The applied algometer pressure is mathematically transformed into a logarithmic scale of sensitivity levels similar to the Decibel scale, which uses air pressure threshold for the determination of hearing thresholds. Algometric Measure (PT) = Log 200 - Log 100 (100 - threshold in kilogram/14 kilos)) (PT unit, also termed "1 ballegard" or "1 bal" herein). An increase in 30 PT units corresponds to a 100% increase in sensitivity (i.e. lower pain threshold). The scale ranges from 0 to 99, with 0 indicating the highest measurable threshold (i.e. 14 kilograms) and 99 indicating the lowest measurable threshold (i.e. 0.5 kilograms) allowing the instrument to measure a 800% increase in pressure pain threshold.

In order to prevent damage to the skin, an alarm sound was activated when pressure reached a level of 14 kilograms.

A special rubber measurement foot plate (having a contact surface area of about 1 cm x 1 cm) was developed with the aim of allowing the determination of pressure pain threshold on the bone without applying noxious stimulation to the skin - as such stimulation might lead to determination of skin pressure pain threshold instead.

For clinical use, PT measurement values < 15 were considered normal for men and values < 30 for women.

The phrase "sympathetic tone-neutral point" denotes a point on or in the body in which the sensitivity to an applied stimulation, such as pressure, is *independent* of the activity level of the sympathetic nervous system. Also covered by the expression is a point on or in the body where increased sympathetic tone causes a higher threshold for sensitivity or nociception in said point or a point, which may show increase in sensitivity, but below the threshold, which is ethically applicable (i.e. 14 kilogram - see above).

The phrase "sympathetic tone-dependent point" denotes a point on or in the body in which the sensitivity to an applied stimulation, such as pressure, is dependent on the activity level of the sympathetic nervous system, in the sense that increased sympathetic tone causes the point to exhibit a lowered threshold for sensitivity and/or nociception.

"Resting conditions" are defined as conditions, at which the individual is at physical and psychological rest, *i.e.* very much the same conditions that should be applied when determining the resting pulse of an individual - *i.e.* conditions where the body is at rest and where the environment is quiet and calm and therefore non-stressing for the individual. One such example for standardized use is the sleep during one night or similar.

A "defined psychological workload" is in the present context a predefined and reproducible set of psychological challenges designed to mirror a psychological workload (mental, cognitive or emotional), which the individual could face in real life. For instance, the defined psychological workload can be in the form of psychological tasks having an increased complexity over time optionally combined with an increasing environmental challenge in the form of e.g. increasing noise, frequent interruptions, increasing time pressure etc. The person of skill in psychology will be able to design tests which gauge any one of the types of workload (mental, cognitive or emotional) which are relevant for the subject being tested. In military training, e.g. it is not uncommon to test the soldiers' ability to withstand increasingly complex and demanding situations, in civil life the use of e.g. airplane simulators and other tests of mental coordination have been in use for decades, and also many computer games involve increasing challenges for the players - these types of challenges are all well suited to gauge the tested subject to "the limits".

The time $\Delta t$ depends on several parameters, but under standardized conditions and if the individual has been subjected to a standardized "hard" psychological challenge, $\Delta t$ will be of a higher value than if the same individual has been subjected to a less hard challenge. Formula I compensates for this by dividing $W_p$ with $\Delta t$.

As an alternative to use $\Delta t$, it is possible to devise different or supplementary embodiments, where $\Delta t_{inv}$, i.e.

the time it takes from a resting PFI measurement to reach a defined level under a given psychological workload, replaces $\Delta t$ in the methods of the invention detailed above. In these embodiments formula I provides a measurement of the "alertness" of the brain, and can be used to supplement the measurement for capacity.

The value $W_p$, which is a characteristic of a given psychological challenge, is arbitrary, but should be calibrated and standardized in order to allow reproducibility, especially in cases where several challenges, such as challenges requiring increasing psychological workload, are used. One simple way to determine the value of $W_p$ for a standardized challenge is to correlate it with a known indicator of stress or sympathetic tone in a number of individuals and then assign a given psychological workload a value relative to other psychological workloads tested in the same persons. This allows for tailoring of challenges with verified and quantified differences in complexity. For the purposes of the present invention it is preferred to define $W_p$ for a number of different psychological challenges, using formula I as the means for assigning values to $W_p$: Since a person does not change his or her $C_{pw}$ within a very short period of time, formula I should ideally provide for the same values of $C_{pw}$, irrespective of the challenge $W_p$, when a person is tested. This means that if the same group of persons within a short while is subjected to a number of different challenges assumed to have different $W_p$, the result of formula I should be constant for each person. So, accordingly, it is possible to simply assign arbitrary values to a number of predefined psychological challenges for each person and also to use the results from all tested persons to arrive at a standardized $W_p$ value for each psychological challenge (convenient when comparing the psychological fitness with other persons).

As an alternative or supplementary test for the above determination of $\Delta t$, the response to a standardized dose of sensory stimulation may be employed, in which case the increase in pain threshold during a predetermined time period (i.e. minutes) is measured. This may be supplemented by a recording of the ability to sustain the obtained increase in pain threshold during a subsequent restitutional period (i.e. minutes).

If the objective of the present invention is to only follow the psychological fitness development of one single person, the person's $C_{pw}$ can initially be set to any arbitrary number (e.g. 100) and similarly, the initial challenges used may be attributed $W_p$ values accordingly so that formula I provides $W_p = 100$ in the initial tests with each challenge. After this initial step, the person's development is then followed using the values obtained for the different challenges.

The monotone function may include multiplication of formula I with a gender adjustment factor and/or the monotone function may include multiplication of formula I with an age adjustment factor. Both these options take into consideration that the capacity for different types of psychological challenges may vary according to age and according to sex. Typically, the value 1 of both these adjustment factors is set relative to the best performing sex or age group based on statistical data in order to compensate for natural variations - if, for instance, elderly normal persons on average exhibit a lower psychological fitness, this may be adjusted by multiplying with a factor which ensures the average performance of each age group is the same. The result is that it becomes possible to compare the psychological fitness directly between different age groups or sexes - in this embodiment of the invention, the PFI is thus used in a manner similar to the IQ, which is, at least for children, age adjusted.

Alternatively, the factors may be omitted in Formula I and in that case it may be convenient to establish the values of at least "average", "below average" and "beyond average" psychological fitness for different age groups and the two sexes.

As mentioned above, the sympathetic tone levels are conveniently determined by a method comprising determining the pain threshold in at least one sympathetic tone dependent location on or in the body. The various means and methods for performing such determinations are provided in the patent applications referenced above.

Another aspect of the invention relates to a method for monitoring the development of capacity for psychological workloads in an individual, said method comprising repeatedly determining the capacity for psychological workload according to the method of the first aspect of the invention detailed above. Such repeated determinations may be made at any interval, but typically repeated determinations are performed at intervals of at least one day, such as at least 2, at least 3, at least 4, at least 5 and at least 6 days. The intervals may be longer, *i.e.* intervals counted in weeks or in months. It is to be noted that the intervals need not be regular.

When over time monitoring a person's capacity for psychological workload, it also becomes possible to incorporate knowledge about the person's psychological and physical state in order to determine if there are specific situations or circumstances which have a negative impact on the psychological fitness - in turn this allows the person to actively avoid such situations or circumstances or, alternatively, learn to manage such situations or circumstances productively. Hence, in order for this to be effective, a practical approach is to maintain a diary or other record of the daily life of the monitored person.

A third aspect of the present invention is related to the above and concerns a method for increasing the capacity for psychological workload in an individual, the method comprising determining an initial value for the capacity according to the method of the first aspect of the invention, subsequently subjecting the individual to measures which reduce the individual's sensitivity to factors which induce psychological stress and monitoring the development in the individual's capacity for psychological workload according to the method of the second aspect of the invention at least until a significant decrease in the value of $\alpha$ and/or a significant increase in the value of At has been observed.

Measures, which may be used to increase the capacity can be selected from a variety of known psychological methods, cognitive methods, meditation techniques, physical exercises, sensory stimulation techniques, and simple planning of daily life. Further, the intervention methods reported in WO 2005/084529, WO 2006/092146, and WO 2008/028976, as well as in US provisional patent application No: 61/036,385, which aims at reducing stress are all applicable in this third aspect of the invention.

A fourth aspect of the invention utilises the above-discussed methods when it is e.g. desired to set up a team (*i.e.* group of persons) which are to perform a task which requires optimum psychological performance of the team as a whole.

This aspect entails establishing the composition of a team of individuals for a task, the method comprising determining, for a number of individuals, which are potential members of the team, the capacity for psychological workload according to the method of the first aspect of the invention and subsequently composing the team based on an evaluation which incorporates the individuals' capacity for psychological workload values. Thus, depending on the structure of the team to be set up, the method may be used to simply exclude members which are not psychologically fit "on the day", meaning that the method is used to set the overall composition of the team, and/or the method may be used to allocate subtasks to the individual members of the team according to their present psychological fitness, meaning that individual roles on the team are assigned as a consequence of the determinations of psychological fitness. The latter option is e.g. convenient if the team has several roles, where not all require high psychological fitness of the same type (some roles may e.g. require or benefit from a high emotional fitness, whereas other roles require a high degree of cognitive fitness).

It goes without saying that the possibility of incorporating the psychological fitness when setting up a team for a sporting event, a demanding managing task, a military operation or any other task where the psychological capacity is of high importance together with physical fitness ought to provide an edge compared to the situation where these psychological features cannot be gauged.

In yet a further aspect, the invention relates to a method for assessing the capacity of an individual or a group of individuals for performing a task, comprising determining, in said individual or in each individual of said group of individuals, the capacity for psychological workload according to the method of the invention as described above. As will appear from the example below, it has been demonstrated that overall performance (*i.e.* performance requiring both physical and psychological capacity) correlates with the psychological workload capacity measurements of the invention.

It is hence pragmatic to include the measurements of the present invention in an integrated evaluation of performance capacity, when an individual or a group of individuals are to perform a task - this allows for both optimization of performance and for accurate prediction of performance. Thus, one embodiment of this aspect entails integration of the result of the inventive determination of psychological workload capacity with at least one further result of a determination of a parameter relevant for performance in said task. Such a parameter is typically related to an individual's physical performance, such as physical strength/force, physical endurance, physical agility, physical condition (e.g. nutritional state, age etc), physical speed (e.g. reaction speed), physical alertness, and physical health.

In one embodiment, the assessed capacity for performing the task is compared to at least one previous assessment - this allows for establishment of a baseline value (or at least an index value), so that it becomes possible to follow the progression of the overall performance capacity. Alternatively, a general standard may be established for use as reference, *i.e.* an individual is chosen as the standard against which other individuals are gauged.

In one embodiment, the value of a determination of capacity for psychological workload calculated on the basis of pain threshold measurements in sympathetic tone dependent points on or in the body is correlated with the assessed capacity for performing the task, meaning that a high pain threshold indicates a higher capacity than a low pain threshold. In other words, the above discussed measurements of psychological workload (when measured by determining pain sensitivity) are used directly as indicators of capacity for performing a task which is not merely one that requires psychological capacity.

The tasks are typically those which are demanding on the individuals involved - examples are competitions in sports, military operations, but also negotiations or other types of work tasks that require a high level of alertness may benefit from the method, because it becomes possible to determine the chances of success in performing the task. So, the task may be a single event, but the task may also take place over a prolonged period of time of more than 24 hours (such as several days, or even several weeks or months), either as a continuous task or as a set of repeated similar or identical partial tasks. Many sporting events require that the athletes are fit (both physically and mentally) during many rounds of competition, and the same is true for other tasks discussed herein. Since the present Example (cf. below) indicates that the method of the invention can also provide an indication of stability in performing a continuous task or set of repeated similar or identical partial tasks, it is preferred that the assessment of the capacity for performing the task also provides an indication of stability in performing a continuous task or a set of repeated similar or identical partial task by an individual or group of individuals.

Finally, since the present invention also allows for improving the capacity for psychological workload (cf. above), it is according to the present invention an important embodiment to improve or increase the capacity of for psychological workload according to the invention in order to improve the overall capacity for performing a task.

To conclude, the present invention enables the optimization of work performance in a large number of situations. By including an accurate assessment of psychological (and thereby total) work capacity together with traditional assessments of physical work capacity, and further by enabling intervention which may improve the psychological work capacity, it is now possible to more accurately optimize performance by a an individual or a group.

These findings also enable a computer system for determining the capacity for psychological workload, or for determining the capacity for performing a task, in a subject or a group of subjects, said system comprising

- a computer readable memory loaded with physiological data for at least one individual,

- an input device for entering values $\alpha$, $\beta$, At as defined above and also for entering value $W_p$ as defined above in the event the system accepts more than one value of $W_p$ - in some embodiments, the system is simple and operates with only one value for $W_p$, whereby it is unnecessary to input it,

- a processor programmed to calculate the capacity for psychological workload based on $\alpha$, $\beta$ and $\Delta t$, and

- an output device for outputting a representation of the capacity for psychological workload calculated on the basis of $\alpha$, $\beta$ and $\Delta t$.

Such a system is also part of the present invention. The physiological data are typically values/characteristics selected from age, gender, body height and weight, various health status parameters, IQ, and other values which are useful for determining the theoretical maximum performance of a given individual. Thus the physiological data provide an indication, which allow the calculation to be correlated to an individual's overall potential for performing a task.

It is convenient if the computer system according further includes a computer readable memory configured to store previous and future values calculated by said processor. This allows the user to not only get a here-and-now picture of the psychological fitness/task capacity but also to study whether training and intervention has any effect. In any event, if the user's data provides for calculations that indicate deterioration, the system may further include an output device for outputting instructions for intervention in the event a calculated capacity for psychological workload exceeds a threshold value. Such intervention may be any type of intervention used to reduce the level of stress, cf. above.

In certain embodiments, the computer system outputs the psychological workload capacity as the result of formula I: $(\beta-\alpha) / (Wp \times \Delta t)$ or as any monotone function thereof, wherein the value of $W_p$ is either derived from the input or is a constant stored in said computer system (cf. the above considerations with respect to one or multi-value $W_p$ systems). The monotone function will in certain embodiments be adjusted as discussed above by multiplying with an age and/or gender adjustment factor.

The computer system may be implemented on a single computer, *i.e.* by implementing a software product on a computer, but in a preferred embodiment the input device and said processor are located on separate computers. This enables a client-server solution or most preferred a solution where the separate computers are connected via the internet and where a user can log on to the system and input data from a distant location, while storage and processing of data are performed on an internet server.

Also contemplated are computer program products which, when read into a computer memory can adapt a computer or a network of computers to function as the above described computer system.

Finally, it is important to note that the present inventor has found that the herein disclosed psychological fitness also serves as a useful indicator in diagnosis, prognosis and treatment follow-up for a number of diseases. It has thus been found that the psychological fitness may be used analogously to the measurements of warning system sensitivity, sympathetic tone and level of stress as discussed in the above-discussed patent applications WO 2005/084529, WO 2006/092146, and WO 2008/028976, as well as in US provisional patent application No: 61/036,385. to provide for prognosis, diagnosis and treatment follow-up and evaluation for the same set of diseases and conditions specified in these patent applications.

EXAMPLE

During 3 months, two elite sailing athletes were followed carefully by constant awareness to their stress level prior to their participation in the Olympic Games. The measurements according to the present invention were used as biofeedback marker for the status of stress level and their ability to obtain allostasis. An intervention program involving both professional treatment as well as self care was employed with the aim to reach a low resting measurement and an optimal allostatic response at the Olympic Games.

Accordingly, up to and during the sailing competitions in the 2008 Olympic Games in Beijing, the following observations were made in a follow up study of the performance of the Danish Olympic gold medallists in the 49'er sailing competition:

The level of stress as well as the adaptive ability was determined from PT measurements as described above. The measurements were conducted with the subjects in supine position; initiated with two measurements on the control point (*i.e.* not sympathetic tone dependent), the dorsal part of the middle phalanx on the left index finger, during which the technique and procedure was introduced. The measuring device was applied with a gradually increase of pressure, in total allowing 3-4 seconds pressure time. The subject was instructed to say "Stop" as soon as discomfort was felt. If the researcher observed a withdrawal reflex, typically the startle reflex from the eyes, this was considered as a stop signal as well. Subsequently, measurements on the sternum (*i.e.* a sympathetic tone dependent point) were conducted following the same procedure. The point for measuring (active point) was identified by palpation of the observer as the most tender point of the skin on the sternum within the area between the third, fourth and fifth intercostal space reflecting the area of segmental innervations of the heart.

The subjects were also instructed to do self-measurements. In previous studies we have demonstrated, that the PT measurement performed by health-professionals has a high precision, r= 0.97, p< 0.0001, n=181 repeated measurements. Non-professionals measuring their own stress level also demonstrated a high precision: r= 0.95, p< 0.0001, n=33.

A PT measure of < 30 is considered normal or low, and a measure > 60 is considered clearly elevated.

Two healthy male athletes aged 26 and 29 years were followed the last 3 months before the Olympic Games in Beijing, 2008. They were elected to represent Denmark in the sail sport competition class 49'er. At the time of election to the Olympic Games these two athletes had several times ranked top 10 in international regattas, but never better than 6th place, during their past 4 years of teamwork.

The two athletes were introduced to an intervention program, which on a personal level involved cognitive exercises, yoga and acupressure, and on a professional level acupuncture (specifically developed for the purpose of recovering allostasis) both using the herein describe PT measure as a guide for demand of intervention.

Treatment commenced in May 2008 (baseline) and continued on a regular basis until June 2008, where the sailors participated in the "Kieler Woche" sailing regatta. After Kieler Woche self-measurements and self-care continued at an increased level with a daily program for lowering the PT measurements and gain full allostasis. This continued until the Olympic Games in august 2008. During competition (Kieler Woche and the Olympic Regatta), measurements by health-professionals and subsequent individualized treatment were performed both before and after the daily races.

Assessment of performance stability:

Nineteen boats participated in the Olympic Regatta. Data from the best 10 boats in the overall standings were used for the following calculations with respect to calculating the stability of performance. We recorded all results from these boats as final position when reaching goal in the individual races among all participants. The accumulated position for the Danish boat was compared to the accumulated mean of position for all top 10 boats. This calculation was also performed for the race at Kieler Woche as well as for the World Championship of 2008. Any difference in slope between the two curves illustrates the frequency of having a position higher than 10 when reaching goal for the Danish boat compared to the mean of all the boats in top ten.

For statistics SPSS 13.0 for Windows was used. A one-way ANOVA was used as statistical analysis for comparison. Non-parametric Pearson analysis was used for correlation analysis.

Results

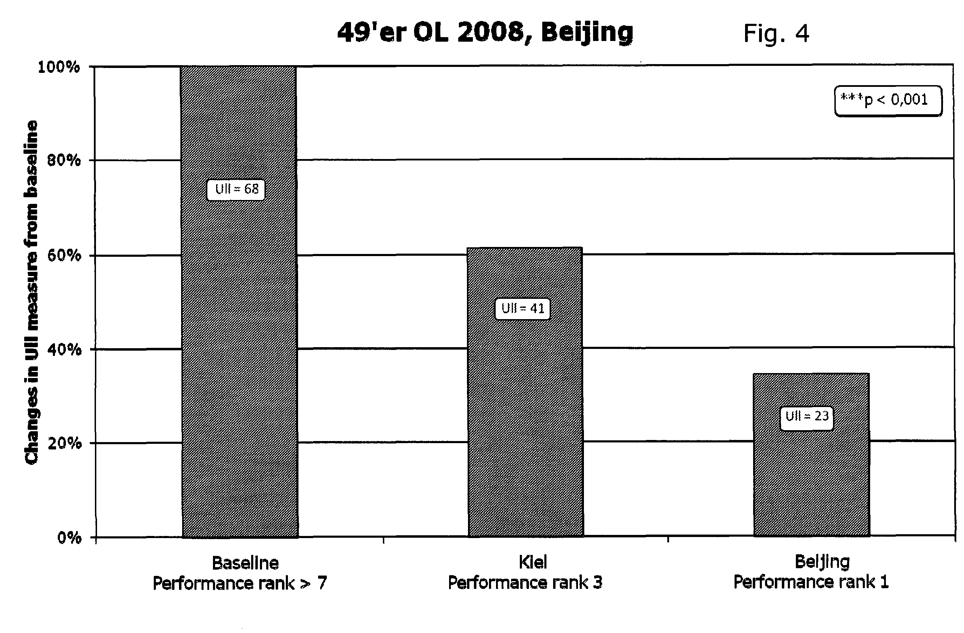

Baseline morning PT measurements, May 2008 were mean 83 for the 2 athletes indicating a situation of elevated resting PT measure, compared to mean 56 during the Kieler Woche, and mean 39 during the Olympic Games (Fig. 4). With respect to overall performance, their best result in 2008 before baseline was number 8 (World Championships 2008), compared to number 3 in Kieler Woche and number 1 at the Olympic Games. These final positions in the 3 races as a measure of performance correlated with the PT measurements, r = 0.9 (p < 0.01). Further, the steady decrease in PT measurements from May to august 2008 was statistically significant (p< 0.001).

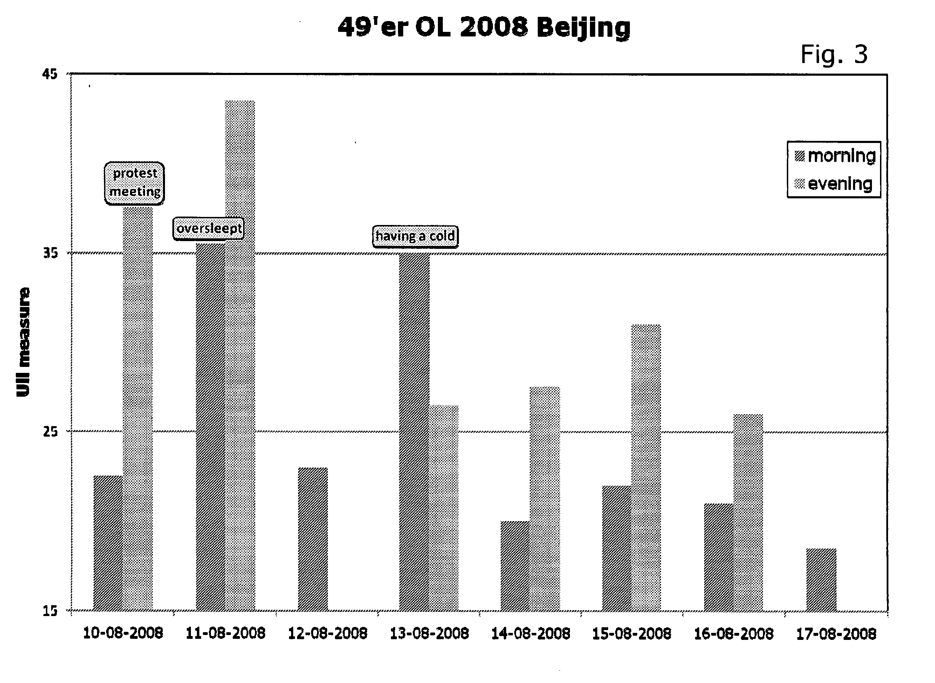

The morning (before race) and evening (before going to sleep) PT measurements during the daily races at the Olympic Games are presented in Fig. 3. The figure shows a median reduction of 18% of evening to morning measure, with the highest reduction of 35%. Having lower morning value than the evening before were found in 6 out of 6 days (p=0.02). So, as indicated, the two athletes exhibited a consistent low level of pain sensitivity (*i.e.* low level of stress) in the morning, unless further factors played a role (a cold incidence and one incidence of oversleeping).

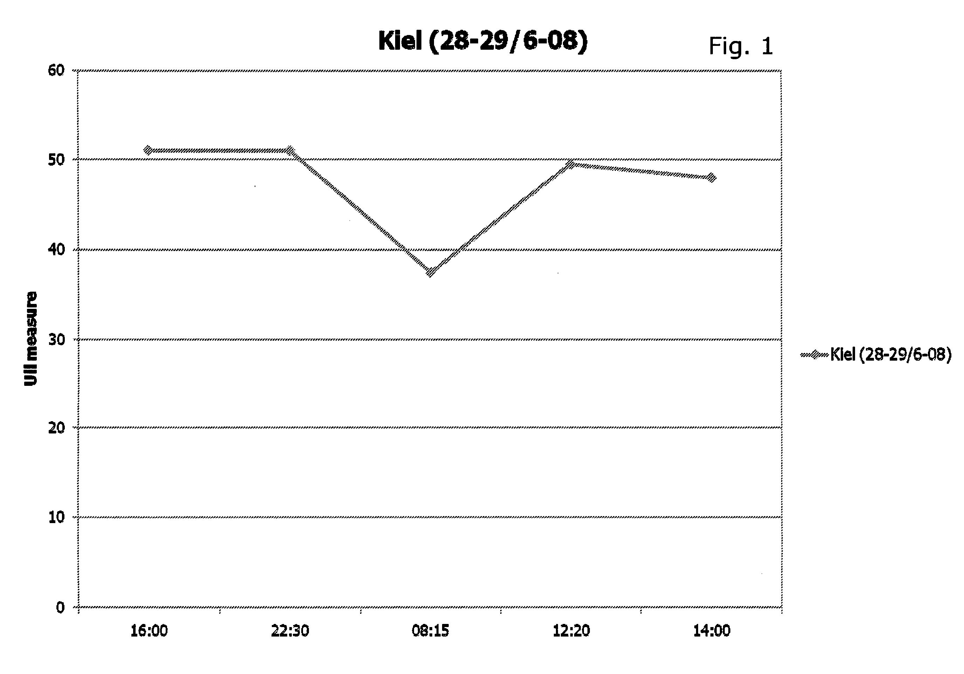

Fig. 1 shows the results of a typical pain threshold measurement in sympathetic tone dependent points in the two athletes during competition in Kiel prior to the Olympic Games. Generally, the measurements indicate a high pain sensitivity (*i.e.* a low threshold), indicating that the complete physiological potential is not sufficiently exploited. Further, the measurements do not change between 4 pm and 10.30 pm, indicating that the "elasticity" is suboptimal - in plain words, the athletes do not seem to reach a psychological rest level during this period. Nevertheless, during sleep at night, the pain sensitivity drops, indicating that sleep is effective and that some elasticity is present. Finally, up to competition after 8 am, there is an increase in pain sensitivity, coupled to an increase in performance capacity, but after competition, the reduction in sensitivity is minor, indicating that psychological fitness could be better.

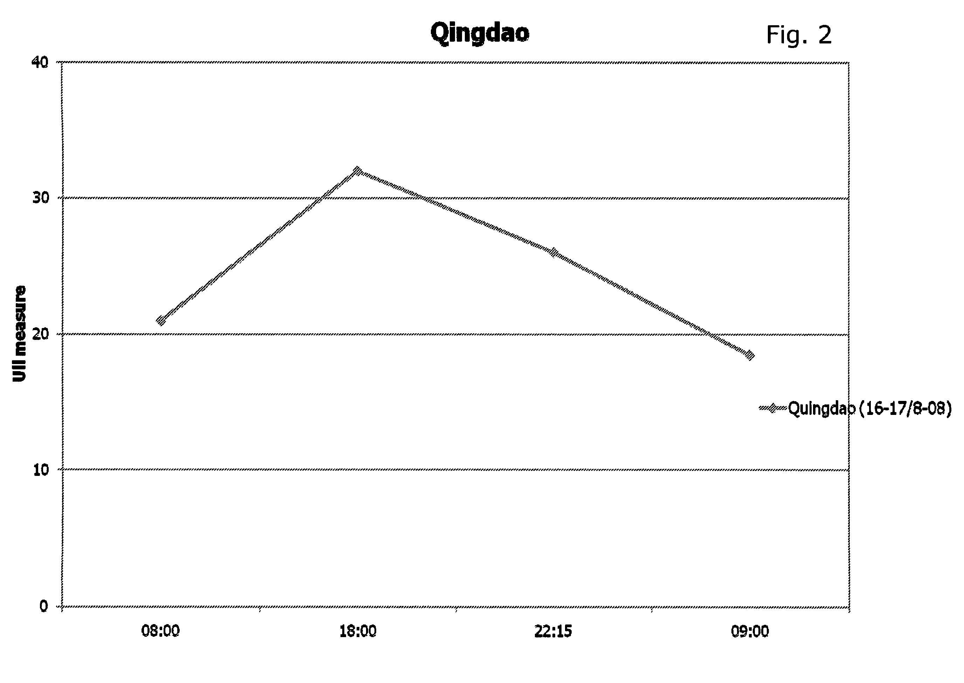

Fig. 2 shows a curve corresponding to Fig. 1, but from the Olympic Games 2008. It is noteworthy that the general level of pain sensitivity is considerably lower, and that the reversion to the resting (night) level is initiated immediately after end of competition. Overall, the measurements indicate a higher level of psychological fitness.

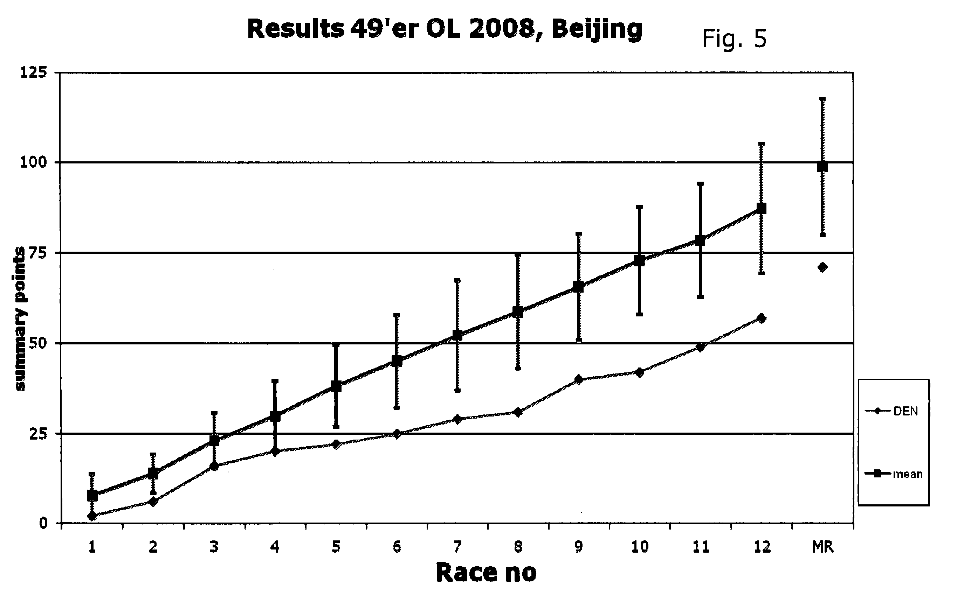

Concerning performance during the races, we investigated the mean (+/- standard deviation) for the top 10 boats among all participants (N = 19 for the Olympic competition), compared to the position of the Danish boat, cf. Fig. 5. The difference in slope for the Olympic Game sailings illustrates that among the other 9 boats in top 10, several boats had positions > 10 in each race, while the Danish boat stayed within the 10 best boats in each and every race. Furthermore, the Danish boat had a stability in sailing similar to the rest of the top ten boats in Kiel (data not shown), while had a stability inferior to the other boats in top 10 for the VM 2008 (data not shown), thus suggesting an increase in performance stability for the Danish boat, when compared to the top 10 boats in general (correlation coefficient between improvement in slope for the Danish boat and decrease in PT measurement value ($r = 0.9$; $p < 0.01$).

Discussion:

The present study suggest that pressure pain sensitivity of the sternum is correlated to the overall performance in top sports, and that it is feasible to include a biofeedback guided stress management program in addition to an existing high preparation load. Activation of the sympathetic tone increases the sensitivity in specific cutaneous sensor cells on the sternum and can be used as a biofeedback marker for measurement of current state of stress level. The dynamic process of obtaining allostasis is of great importance for making top performance in sports, as it is diminished in persistent stress which manifest as negative effects on performance. In the present study we measured allostasis using a newly developed instrument, which measures the pressure pain sensitivity of the polymodal nociceptors on the sternum with great precision. Until now the term allostasis presents a theoretic model for stress and currently there exists no consensus on stress measurement. This seems however possible by using the PT measurement described herein. Furthermore, the PT measurement level can be obtained with high precision by self-measurements, which gives the method potential use in many situations, in which a biofeedback guided treatment of and coping with stress is wanted.

We tried to obtain allostasis in two athletes participating in the Olympic sailing regatta. The daily use of the PT measurement in combination with the intervention program resulted in a steady decrease in PT measurement values from clearly elevated levels of mean 83, to a significant lower level of mean 39.

The methodology was included in a stress management program and added to the palette of tools supporting the athletes performing their sailing sport. The observed decrease in stress level, measured as decrease in pressure pain sensitivity of the sternum correlated to a constant improvement in performance, from the World Championship through the Kieler Woche to the Olympic Games (PT measurement values versus final position in the 3 races, r= 0.9, p< 0.001), demonstrating that the program applied did make sense. The athletes won the Gold Medal, which was remarkable when taking into account that the athletes just 3 months ahead of the Olympic Games never had performed better than a 6th place in international regattas.

During the Olympic Regatta a rather constant level of both morning and evening measurement values was observed with as expected higher levels in the evening due to daily competitive activities with higher activation of the sympathetic tone compared to lower levels in the morning due to activity of the parasympathetic tone during the night. Deviation from this pattern was found on days with protest meeting, sleep over and the catch of a cold. Thus it appears that stressors forced on the athletes increased their PT measurement values beyond the general level. We postulate that if no action was taken to reduce the level of stress, tension and anxiety would have build up reducing the athlete's performance. On days with these external stressors, we carefully intervened with the intention of bringing down their PT measurement values to the general level of morning and evening. Since both measurement and acupressure as self-treatment, was done at the same spot on the sternum, it can be argued that adaption of the nociceptors - the modular basis for measurement of the pressure pain threshold - could have occurred. However, as can be seen (Fig. 3) the viability of the nociceptors were still very much intact as the PT measurement values could increase up to 40-50 % of morning or evening baseline at times where the athletes were challenged with stressors.

A predefined hypothesis was, that ensuring allostasis before and during the Olympic Regatta would result in a more constant series of results in the races, since the athletes, due to intervention would be concentrated to the same top level every day. Fig. 5 demonstrates that this was the case: The accumulated variability in the daily positions was lower for the Danish team than for the other top 10 crews, *i.e.* the slope in figure 3 was lower for the Danish boat during the Olympic Games, when compared to the mean of the other 10 sailors. Furthermore, this index of variability as compared to the historical results during the World Championships 2008 and Kieler Woche improved constantly for the Danish boat, when compared to the mean of the top 10 boats, and this improvement correlated closely to the simultaneous improvement in final positions *and* the PT measurement values.

Our findings support the model of allostasis and the allostatic load; i.e. the beneficial effect from the ability to overcome transient stress, as well as the persistent stress, the allostatic load. These terms might be compared to physical fitness: High physical fitness is associated with low resting pulse rate, and the ability for the heart to increase workload and pulse rate sufficiently, as well as the ability to quick recovery. Similarly, the present study suggests, that allostasis as showed in Fig. 3, and high performance is linked to a low resting PT measurement value, while loss of allostasis and inferior performance is associated with a high resting PT measurement value. This comparison is in line with the finding, that the PT measurement value seems positively correlated to resting heart rate and work capacity of the heart.

In order to distinctly describe this expansion of the understanding of allostasis and allostatic load, we may suggest the introduction the above described psychological fitness, where a high psychological fitness is associated with 1) a low resting PT measurement value, 2) a fast increase in PT measurement values during a challenge or task and a similarly fast recovery, when the challenge is over, and 3) the ability to react with only minor increase in PT measurement values during disturbing non-treating sensory input. In line with this, a low psychological fitness has the opposite characteristics.

In conclusion, the findings suggest that there is a correlation between the pressure pain sensitivity of the sternum and overall performance. Furthermore, that the inclusion of biofeedback stress management program is feasible in top performance sports and other demanding tasks and may have the ability to improve allostasis - and thus re-establish full adaptive capacity, a finding which preferable could be tested in a randomized study.

**Claims**

1. A method for determining the capacity for psychological workload in an individual, comprising

   (a) determining the level of sympathetic tone in said individual under resting conditions ($\alpha$),
   (b) determining the level of sympathetic tone ($\beta$) in said individual under a defined psychological workload ($W_p$);
   **characterized in that** the method further comprises:
   (c) determining the time ($\Delta t$) it takes said individual to revert from the sympathetic tone level $\beta$ under the defined psychological workload $W_p$, to the sympathetic tone level $\alpha$ under resting conditions, and
   (d) expressing the capacity for psychological workload as a function of $\beta$, $\alpha$ and At.

2. The method of claim 1, wherein step (d) entails calculating the psychological workload capacity according to the formula I:

$$(\beta\text{-}\alpha) \, / \, (W_p \times \Delta t) \text{ or any monotone function thereof.}$$

3. The method according to claim 1 or 2, wherein the monotone function includes multiplication of formula I with an age adjustment factor and/or with a gender adjustment factor, such as a positive number, which based on statistical data allows direct comparison of values for psychological workload capacity between different age groups and/or the two sexes.

4. The method according to any one of the preceding claims, wherein sympathetic tone levels are determined by a method comprising determining the pain threshold in at least one sympathetic tone dependent location on or in the body.

5. The method according to any one of the preceding claims, wherein $\beta$ is determined after a stress-inducing intervention or after a stress-reducing intervention, such as a standard dose sensory stimulation.

6. The method according to any one of the preceding claims wherein the psychological workload is selected from the group consisting of mental workload, emotional workload and cognitive workload.

7. A method for monitoring the development of capacity for psychological workloads in an individual, said method comprising repeatedly determining the capacity for psychological workload according to the method of any one of the preceding claims.

8.    A method for establishing the composition of a team of individuals for a task, the method comprising determining, for a number of individuals which are potential members of the team, the capacity for psychological workload according to the method of any one of claims 1-6, and subsequently composing the team based on an evaluation which incorporates the individuals' capacity for psychological workload values.

9.    A method for assessing the capacity of an individual or a group of individuals for performing a task, comprising determining, in said individual or in each individual of said group of individuals, the capacity for psychological workload according to the method of any one of claims 1-6, and optionally integrating the result of the determination with at least one further result of a determination of a parameter relevant for performance in said task, such as a parameter relating to an individual's physical performance.

10.    The method according to claim 9, wherein the assessed capacity for performing the task is compared to at least one previous assessment, such as a previous assessment, which is used as a standard.

11.    The method according to claim 9, wherein the value of a determination of capacity for psychological workload calculated on the basis of pain threshold measurements in sympathetic tone dependent points on or in the body correlates with the assessed capacity for performing the task, meaning that a high threshold indicates a higher capacity than a low threshold.

12.    A computer system for determining the capacity for psychological workload in a subject or a group of subjects or for determining the capacity for performing a task, said system comprising

      - a computer readable memory loaded with physiological data for at least one individual,
      - an input device for entering values $\alpha$, $\beta$, At as defined in claim 1 and also for entering value $W_p$ as defined in claim 1 in the event the system accepts more than one value of $W_p$,
      - a processor programmed to calculate the capacity for psychological workload based on $\alpha$, $\beta$ and $\Delta t$, and
      - an output device for outputting a representation of the capacity for psychological workload calculated on the basis of $\alpha$, $\beta$ and $\Delta t$.

13.    The computer system according to claim 12, further including a computer readable memory configured to store previous and future values calculated by said processor and/or further inluding an output device for outputting instructions for intervention in the event a calculated capacity for psychological workload exceeds a threshold value.

14.    The computer system according to claim 12, wherein said computer system outputs the psychological workload capacity as the result of formula I: $(\beta-\alpha) / (W_p \times \Delta t)$ or as any monotone function thereof, wherein the value of $W_p$ is either derived from the input or is a constant stored in said computer system, where the monotone function optionally is as defined in claim 3, and wherein the values of $\beta$ and $\alpha$ optionally are as defined in claim 4 or 5.

15.    The computer system according to any one of claims 12-14, wherein said input device and said processor are located on separate computers, such as separate computers connected via the internet.

**Patentansprüche**

1.    Verfahren zum Feststellen der Fähigkeit zum Bewältigen einer psychischen Belastung in einem Menschen, das Folgendes umfasst:

      (a) Feststellen des Grades des Sympathikotonus in dem Menschen im Ruhezustand ($\alpha$),
      (b) Feststellen des Grades des Sympathikotonus ($\beta$) in dem Menschen bei einer definierten psychischen Belastung ($W_p$);
      **dadurch gekennzeichnet, dass** das Verfahren des Weiteren Folgendes umfasst:
      (c) Feststellen der Zeit ($\Delta t$), die es dauert, bis die Person von dem Sympathikotonusgrad $\beta$ bei der definierten psychischen Belastung $W_p$ zu dem Sympathikotonusgrad $\alpha$ im Ruhezustand zurückkehrt, und
      (d) Ausdrücken der Fähigkeit zum Bewältigen einer psychischen Belastung als eine Funktion von $\beta$, $\alpha$ und $\Delta t$.

2.    Verfahren nach Anspruch 1, wobei Schritt (d) das Berechnen der Fähigkeit zum Bewältigen einer psychischen Belastung gemäß folgender Formel I beinhaltet:

$$(\beta-\alpha) \ / \ (W_p \times \Delta t) \ \text{oder eine monotone Funktion davon.}$$

3. Verfahren nach Anspruch 1 oder 2, wobei die monotone Funktion eine Multiplikation der Formel I mit einem Alterskorrekturfaktor und/oder mit einem Geschlechtskorrekturfaktor, wie zum Beispiel einer positiven Zahl, enthält, was anhand statistischer Daten einen direkten Vergleich von Werten für die Fähigkeit zum Bewältigen einer psychischen Belastung zwischen verschiedenen Altersgruppen und/oder den beiden Geschlechtern erlaubt.

4. Verfahren nach einem der vorangehenden Ansprüche, wobei Sympathikotonusgrade mittels eines Verfahrens ermittelt werden, welches das Feststellen der Schmerzschwelle an mindestens einer Sympathikotonusabhängigen Stelle am oder im Körper umfasst.

5. Verfahren nach einem der vorangehenden Ansprüche, wobei $\beta$ nach einer stressinduzierenden Intervention oder nach einer stressreduzierenden Intervention, wie zum Beispiel einer standarddosierten sensorischen Stimulation, festgestellt wird.

6. Verfahren nach einem der vorangehenden Ansprüche, wobei die psychische Belastung aus folgender Gruppe ausgewählt wird: geistige Belastung, emotionale Belastung und kognitive Belastung.

7. Verfahren zum Überwachen der Entwicklung der Fähigkeit zum Bewältigen psychischer Belastungen in einem Menschen, wobei das Verfahren umfasst, wiederholt die Fähigkeit zum Bewältigen einer psychischen Belastung gemäß dem Verfahren nach einem der vorangehenden Ansprüche festzustellen.

8. Verfahren zum Festlegen der Zusammensetzung eines Teams aus Personen für eine Aufgabe, wobei das Verfahren Folgendes umfasst: Feststellen, für eine Anzahl von Personen, die potenzielle Mitglieder des Teams sind, der Fähigkeit zum Bewältigen einer psychischen Belastung gemäß dem Verfahren nach einem der Ansprüche 1-6, und anschließendes Zusammenstellen des Teams auf der Grundlage einer Evaluierung, welche die Werte für die Fähigkeit der Personen zum Bewältigen einer psychischen Belastung beinhaltet.

9. Verfahren zum Beurteilen der Fähigkeit eines Menschen oder einer Gruppe von Personen zum Erfüllen einer Aufgabe, das Folgendes umfasst: Feststellen, bei der Person oder jeder Person in der Personengruppe, der Fähigkeit zum Bewältigen einer psychischen Belastung gemäß dem Verfahren nach einem der Ansprüche 1-6, und optionales Zusammenführen des Ergebnisses der Feststellung mit mindestens einem weiteren Ergebnis einer Feststellung eines Parameters, der für die Leistung während der Aufgabe relevant ist, wie zum Beispiel ein Parameter im Zusammenhang mit der körperlichen Leistung eines Menschen.

10. Verfahren nach Anspruch 9, wobei die beurteilte Fähigkeit zum Erfüllen der Aufgabe mit mindestens einer vorherigen Beurteilung verglichen wird, wie zum Beispiel einer vorherigen Beurteilung, die als ein Standard verwendet wird.

11. Verfahren nach Anspruch 9, wobei der Wert einer Feststellung der Fähigkeit zum Bewältigen einer psychischen Belastung, der anhand von Schmerzschwellenmessungen an Sympathikotonusabhängigen Punkten am oder im Körper berechnet wird, mit der beurteilten Fähigkeit zum Erfüllen der Aufgabe korreliert, was bedeutet, dass eine hohe Schwelle eine höhere Fähigkeit anzeigt als eine niedrige Schwelle.

12. Computersystem zum Feststellen der Fähigkeit zum Bewältigen einer psychischen Belastung eines Menschen oder einer Gruppe von Personen oder zum Feststellen der Fähigkeit zum Erfüllen einer Aufgabe, wobei das System Folgendes umfasst:

   - einen computerlesbaren Speicher, der mit physiologischen Daten für mindestens eine Person beladen ist,
   - ein Eingabegerät zum Eingeben von Werten $\alpha$, $\beta$, $\Delta t$ im Sinne von Anspruch 1 und außerdem zum Eingeben eines Wertes $W_p$ im Sinne von Anspruch 1, falls das System mehr als einen Wert von $W_p$ akzeptiert,
   - einen Prozessor, der dafür programmiert ist, die Fähigkeit zum Bewältigen einer psychischen Belastung anhand von $\alpha$, $\beta$ und $\Delta t$ zu berechnen, und
   - ein Ausgabegerät zum Ausgeben einer Darstellung der Fähigkeit zum Bewältigen einer psychischen Belastung, die anhand von $\alpha$, $\beta$ und $\Delta t$ berechnet wurde.

13. Computersystem nach Anspruch 12, das des Weiteren einen computerlesbaren Speicher enthält, der dafür konfiguriert ist, frühere und künftige Werte zu speichern, die durch den Prozessor berechnet wurden, und/oder des Weiteren ein Ausgabegerät enthält, um Instruktionen für eine Intervention auszugeben, falls eine berechnete Fähigkeit zum Bewältigen einer psychischen Belastung einen Schwellenwert übersteigt.

14. Computersystem nach Anspruch 12, wobei das Computersystem die Fähigkeit zum Bewältigen einer psychischen Belastung als das Ergebnis der Formel I: $(\beta-\alpha) / (W_p \times \Delta t)$ oder als eine monotone Funktion davon ausgibt, wobei der Wert von $W_p$ entweder aus der Eingabe abgeleitet wird oder eine Konstante ist, die in dem Computersystem gespeichert ist, wobei die monotone Funktion optional wie in Anspruch 3 definiert ist, und, wobei die Werte von $\beta$ und $\alpha$ optional wie in Anspruch 4 oder 5 definiert sind.

15. Computersystem nach einem der Ansprüche 12-14, wobei sich das Eingabegerät und der Prozessor auf separaten Computern befinden, wie zum Beispiel separaten Computern, die über das Internet verbunden sind.

**Revendications**

1. Procédé de détermination de la capacité de charge de travail psychologique d'un individu, comprenant :

   (a) la détermination du niveau du tonus sympathique dudit individu au repos ($\alpha$)
   (b) la détermination du niveau de tonus sympathique ($\beta$) dudit individu soumis à une charge de travail psychologique définie ($W_p$) ;
   **caractérisé en ce que** le procédé comprend en outre :
   (c) la détermination du temps ($\Delta t$) qu'il faut au dit individu pour revenir du niveau de tonus sympathique $\beta$ à la charge de travail psychologique définie ($W_p$) au niveau de tonus sympathique $\alpha$ au repos, et
   (d) l'expression de la capacité de charge de travail psychologique en fonction de $\beta$, $\alpha$ et $\Delta t$.

2. Procédé selon la revendication 1, dans lequel l'étape (d) implique le calcul de la capacité de charge de travail psychologique selon la formule I :

   $(\beta - \alpha) / (W_p \times \Delta t)$ ou n'importe quelle fonction monotone de celle-ci.

3. Procédé selon la revendication 1 ou 2, dans lequel la fonction monotone comprend la multiplication de la formule I par un facteur d'ajustement d'âge et/ou un facteur d'ajustement de sexe, comme un nombre positif, qui, sur la base de données statistiques, permet une comparaison directe de valeurs de capacité de charge de travail psychologique entre différents groupes d'âges et/ou les deux sexes.

4. Procédé selon l'une quelconque des revendications précédentes, dans lequel les niveaux de tonus sympathique sont déterminés par un procédé comprenant la détermination du seuil de douleur à au moins un emplacement dépendant du tonus sympathique sur le corps ou dans le corps.

5. Procédé selon l'une quelconque des revendications précédentes, dans lequel $\beta$ est déterminé après une intervention induisant du stress ou après une intervention réduisant le stress, comme une stimulation sensorielle de dose standard.

6. Procédé selon l'une quelconque des revendications précédentes, dans lequel la charge de travail psychologique est sélectionnée dans le groupe se composant d'une charge de travail mentale, d'une charge de travail émotionnelle et d'une charge de travail cognitive.

7. Procédé de surveillance du développement de la capacité de charges de travail psychologiques d'un individu, ledit procédé comprenant la détermination à répétition de la capacité de charge de travail selon le procédé de l'une quelconque des revendications précédentes.

8. Procédé d'établissement de la composition d'une équipe d'individus pour une tâche, le procédé comprenant la détermination, pour un nombre d'individus qui sont des membres potentiels de l'équipe, de la capacité de charge de travail psychologique selon le procédé de l'une quelconque des revendications 1 à 6, puis la composition de l'équipe sur la base d'une évaluation incorporant la capacité de l'individu pour des valeurs de charge de travail psychologique.

9. Procédé d'évaluation de la capacité d'un individu ou d'un groupe d'individus à effectuer une tâche, comprenant la détermination, chez ledit individu ou chez chaque individu dudit groupe d'individus, de la capacité de charge de travail psychologique selon le procédé de l'une quelconque des revendications 1 à 6, et facultativement l'intégration du résultat de la détermination avec au moins un autre résultat d'une détermination d'un paramètre pertinent pour l'exécution de ladite tâche, comme un paramètre relatif à la performance physique d'un individu.

10. Procédé selon la revendication 9, dans lequel la capacité évaluée à effectuer la tâche est comparée à au moins une évaluation précédente, comme une évaluation précédente servant de référence.

11. Procédé selon la revendication 9, dans lequel la valeur d'une détermination de capacité de charge de travail psychologique calculée sur la base de mesures de seuil de douleur à des points dépendant du tonus sympathique sur le corps ou dans le corps est en corrélation avec la capacité évaluée à exécuter la tâche, ce qui signifie qu'un seuil haut indique une capacité supérieure à celle d'un seuil bas.

12. Système informatique de détermination de la capacité de charge de travail psychologique d'un sujet ou d'un groupe de sujets ou de détermination de la capacité à effectuer une tâche, ledit système comprenant :

- une mémoire lisible par ordinateur chargée de données physiologiques pour au moins un individu,
- un dispositif d'entrée pour entrer des valeurs $\alpha$, $\beta$, $\Delta t$ selon la revendication 1 et également pour entrer une valeur $W_p$ selon la revendication 1 dans le cas où le système accepte plusieurs valeurs de $W_p$,
- un processeur programmé pour calculer la capacité de charge de travail psychologique sur la base de $\alpha$, $\beta$, et $\Delta t$, et
- un dispositif de sortie pour délivrer une représentation de la capacité de charge de travail psychologique calculée sur la base de $\alpha$, $\beta$, et $\Delta t$.

13. Système informatique selon la revendication 12, comprenant en outre une mémoire lisible par ordinateur configurée pour stocker des valeurs précédentes et futures calculées par ledit processeur et/ou comprenant en outre un dispositif de sortie pour délivrer des instructions d'intervention dans le cas où une capacité calculée de charge de travail psychologique dépasse une valeur de seuil.

14. Système informatique selon la revendication 12, dans lequel ledit système informatique délivre la capacité de charge de travail psychologique en tant que résultat de la formule I : $(\beta - \alpha) / (W_p \times \Delta t)$ ou de n'importe quelle fonction monotone de celle-ci, dans lequel la valeur de $W_p$ est dérivée de l'entrée ou est une constante stockée dans ledit système informatique, où la fonction monotone est facultativement selon la revendication 3, et dans lequel les valeurs de $\beta$ et $\alpha$ sont facultativement selon la revendication 4 ou 5.

15. Système informatique selon l'une quelconque des revendications 12 à 14, dans lequel ledit dispositif d'entrée et ledit processeur se trouvent sur des ordinateurs distincts, comme des ordinateurs distincts connectés par l'intermédiaire de l'Internet.

Fig. 1

Kiel (28-29/6-08)

Kiel (28-29/6-08)

UII measure

60 50 40 30 20 10 0

16:00 22:30 08:15 12:20 14:00

**Qingdao**

Fig. 2

Quingdao (16-17/8-08)

EP 2 352 430 B1

**49'er OL 2008 Beijing**

Fig. 3

EP 2 352 430 B1

**49'er OL 2008, Beijing**  Fig. 4

EP 2 352 430 B1

Results 49'er OL 2008, Beijing    Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20020005784 A
- WO 2005084529 A
- WO 2006092146 A
- WO 2008028976 A
- US 61036385 B

### Non-patent literature cited in the description

- **NUSSBAUM, E. L. ; DOWNES, L.** Reliability of clinical pressure-pain algometric measurements obtained on consecutive days. *Phys Therapy,* 1998, vol. 78, 160-169